# EUROPÄISCHE PATENTSCHRIFT

(11) **EP 1 203 559 B1**
(45) Veröffentlichungstag und Bekanntmachung des Hinweises auf die Patenterteilung: **13.11.2002**
(21) Anmeldenummer: 02004504.3
(22) Anmeldetag: 30.08.1997
(51) Int. Cl.: A61B 5/0484, A61B 5/0478, A61B 5/12

(54) **Gerät zur Ableitung akustisch evozierter Gehirnpotentiale**
Device for derivation of acoustically evoked brain potentials
Appareil pour la dérivation de potentiels cérébraux évoqués acoustiquement

(30) Priorität: 07.09.1996 DE 29615656 U
(43) Veröffentlichungstag der Anmeldung: 08.05.2002
(62) Teilanmeldung aus: 97115066.9
(73) Patentinhaber: Finkenzeller, Peter, Prof. Dr. rer. nat., 91054 Erlangen (DE); Seipl, Johann, 81547 München (DE); Kammermeier, Heike, 89438 Eppisburg (DE)
(72) Erfinder: Finkenzeller, Peter, Prof.Dr.rer.nat., 91054 Erlangen (DE)
(74) Vertreter: Gallo, Wolfgang, Dipl.-Ing. (FH)

(56) Entgegenhaltungen:
- EP-A- 0 541 315
- US-A- 4 462 411

## Beschreibung

Die Ableitung akustisch evozierter elektrischer Gehimpotentiale ist ein bekanntes audiometrisches Diagnoseverfahren zur Prüfung des Hörvermögens und zur Beurteilung verschiedener Ursachen von Hörschäden ohne aktive Mitwirkung des Probanden.

Dieses Verfahren wird in der Fachwelt als ERA (Electric Response Audiometry) bzw. BERA (Brainstem Electric Response Audiometry) bzw. Hirnstamm-Audiometrie bezeichnet. Einsatzgebiete dieses Verfahrens sind beispielsweise die Durchführung erster Hörtests bei Neugeborenen, die Prüfung der Hörfähigkeit von Säuglingen oder auch von bewusstlosen Personen, wie z. B. Unfallopfer, und die Diagnose von neurologischen Erkrankungen, z. B. Akustikus-Neurinomen. Auch intraoperative Hörfähigkeitsprüfungen sind mit diesem Verfahren möglich.

Die Auslösung von elektrischen Gehirnpotentialen erfolgt dabei durch akustische Stimulation des Ohres über Luft- oder Knochenleitung. Dazu verwendet man üblicherweise Kopfhörer.

Die dadurch vom Hirnstamm erzeugten elektrischen Signale werden über am Kopf angebrachte Elektroden abgeleitet. Üblicherweise werden drei Elektroden verwendet, nämlich eine Elektrode zur Festlegung des Bezugspotentials und zwei aktive Elektroden zur Ableitung der akustisch evozierten elektrischen Signale an zwei verschiedenen Stellen des Kopfes.

Die akustische Stimulation des Ohres erfolgt dabei in Form von Klick-Reizen oder zur unmittelbaren Bestimmung der Hörschwelle aus einer rasch aufeinanderfolgenden Serie von Klicks mit aufsteigendem Pegel. Der Himstamm erzeugt auf jeden Klick Potentialwellen, die nach Erfassung und Ableitung über die Elektroden gemittelt werden.

Bisher werden die Elektroden üblicherweise angeklebt oder mechanisch oder anderweitig einzeln am Kopf fixiert. Dies ist zum einen zeitaufwendig, zum anderen belastet es den Patienten. Wegen der Kabelverbindungen besteht auch die Gefahr des falschen Zusammensteckens. Außerdem ist die Anordnung elektrischen Einstreuungen durch Fremdfelder unterworfen, was wegen der äußerst kleinen abzuleitenden Signalpotentiale kritisch ist. Hinzu kommt, daß das Ankleben und nachfolgende Wiederablösen der Elektroden an der empfindlichen Haut von Neugeborenen bzw. Säuglingen problematisch ist.

Aus der US-A-4 462 411 ist eine Anordnung mit den Merkmalen des Oberbegriffs des Anspruchs 1 bekannt. Dort umfaßt die Anordnung neben den am Kopf anzubringenden Elektroden einen Kopfhörer mit zwei über einen relativ starren Bügel verbundenen Hörmuscheln.

Der Erfindung liegt daher die Aufgabe zugrunde, ein Gerät zu schaffen, mit welchem eine verbesserte Ableitung der Gehirnpotentiale möglich ist.

Diese Aufgabe wird gemäß der Erfindung durch das in den Ansprüchen angegebene Gerät gelöst.

Bei dem erfindungsgemäßen Gerät sind die Elektroden zu einer in sich relativ starren Anordnung zusammengefasst, die vom Operator mit der Hand an den Kopf des Probanden gehalten oder anderweitig als Ganzes am Kopf fixiert wird, und ein als Reizgeber dienender akustischer Wandler in Form eines Lautsprechers, eines Einsteckhörers, eines Knochenleitungshörers oder einer OAE-Sonde (Sonde für otoakustisch Emissionen) ist mit dem Gerät verbunden. Das Gerät beaufschlagt den Reizgeber mit entsprechenden Signalen, um die zur akustischen Reizung dienende Klickfolge zu erzeugen, und mit der Elektrodenanordnung werden die dadurch evozierten elektrischen Gehirnpotentiale abgeleitet und in dem Gerät ausgewertet. Das erfindungsgemäße Gerät ist insbesondere bei Säuglingen von Vorteil, bei denen die Untersuchung üblicherweise stattfindet, wenn sie schlafen, da insbesondere da Anbringen und die Entfernung der Elektroden keiner besonderen Manipulation bedarf und so die Messung am Probanden, z. B. einem Säugling, innerhalb wesentlich kürzerer zeit als bisher üblich vollständig erfolgt.

Die Zusammenfassung mehrerer Elektroden zu einer in sich relativ starren Anordnung, die als Ganzes am Kopf einer Person angeordnet werden kann, ist an sich aus der US-PS 4 706 679 zum Zwecke der Elektroenzephalographie bekannt Dort ist ein Rahmen mit mehreren federnden Schenkeln vorgesehen, die an ihren Enden Elektroden tragen, du elektrische Gehirnpotentiale zu erfassen. Bei der bekannten Anordnung ist der Rahmen insbesondere dafür vorgesehen, an der Lehne eines Patientenstuhles angebracht zu werden, in dem sich der Patient für das Elektroenzephalogramm hineinlegt. Jedoch liefert dieser Stand der Technik keine Anregung zur Durchführung der Hirnstamm-Audiometrie unter Anwendung derart zusammengefasster Elektroden in Kombination mit einem akustischen Reizgeber und einer zur Reizsignalerzeugung und zur Ableitung und Auswertung der evozierten Hirnstammsignale dienenden Signalgeber/Signalauswerteeinheit.

Vorzugsweise enthält die Elektrodeneinheit auch den als Reizgeber dienenden akustischen Wandler in Form eines Lautsprechers oder eines Knochenleitungs-Schallgebers als damit integrierte Komponente.

Bei dem erfindungsgemäßen Gerät kann die Elektrodeneinheit auch gleich den EEG-Verstärker als angebaute Komponente aufweisen, so daß ein minimaler Leitungsweg von den Ableitelektroden zum EEG-Verstärker gegeben und damit die Möglichkeit der Störpotentialeinstrahlung minimiert ist.

Das erfindungsgemäße Gerät wird nachstehend anhand von Ausführungsbeispielen unter Bezugnahme auf die anliegenden Zeichnungen kurz in seinen wesentlichen Einzelheiten beschreiben. In den Zeichnungen zeigt:
- Fig. 1: eine Ausführungsform des Geräts mit Freifeldlautsprecher, und
- Fig. 2: eine Ausführungsform des Geräts mit Einsteckhörer bzw. OAE-Sonde

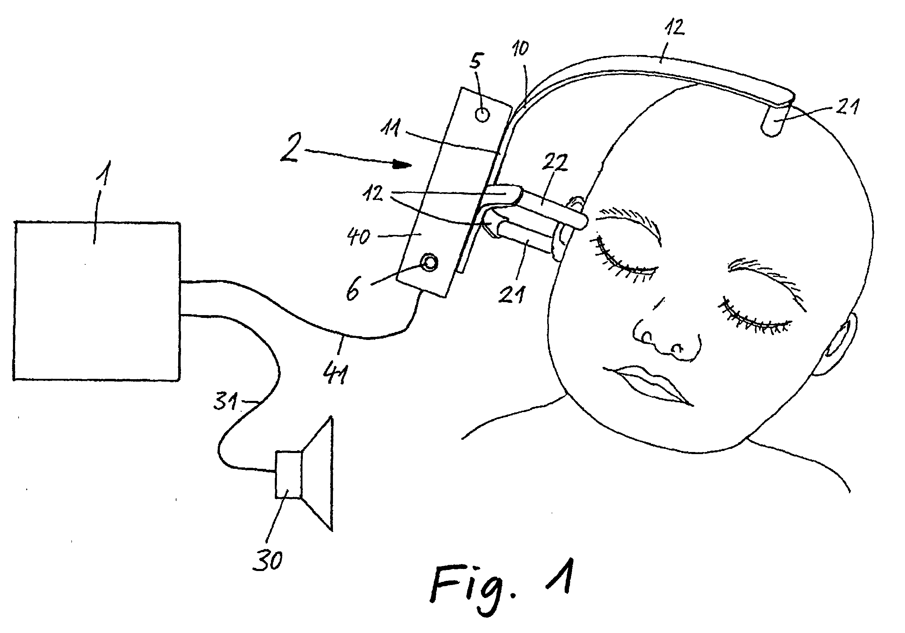

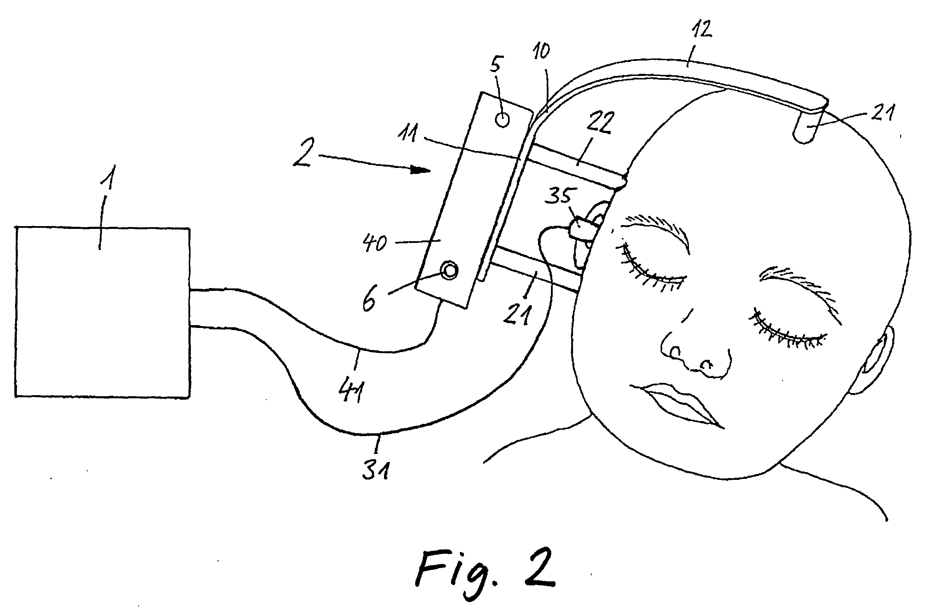

Das Gerät nach den Fig. 1 und 2 besteht aus einer Signalgeber/Signalauswerteeinheit 1, einer Elektrodeneinheit 2 und einem Reizgeber 30 bzw. 35.

Die Elektrodeneinheit 2 besteht aus einem Rahmen 10 mit einem Zentralstück 11 und Armen 12, die Elektroden 21, 22 tragen, und einem ebenfalls am Zentralstück 11 des Rahmens auf dessen von den Elektroden abgewandter Seite angebauten EEG-Verstärker 40.

Ein Kabel 41 verbindet den EEG-Verstärker und somit die Elektrodeneinheit 2 mit der Signalgeber/Auswerteeinheit 1, die außerdem über ein Kabel 31 mit dem Reizgeber 30 bzw. 35 verbunden ist.

Die Signalgeber/Auswerteeinheit 1 erzeugt und überträgt elektrische Reizsignale zum Reizgeber 30 bzw. 35, die dieser in akustische Klickfolgen umsetzt und abstrahlt. Die an den Kopf des Probanden gehaltene Elektrodeneinheit 2 nimmt die vom Hirnstamm evozierten Potentiale auf, die dann, durch den EEG-Verstärker 40 verstärkt, an die Signalgeber/Auswerteeinheit 1 weitergeleitet und dort ausgewertet werden.

Die Elektrodeneinheit 2 weist drei Elektroden 21 bzw. 22 auf. Eine davon dient als Bezugselektrode 22.

Bei der Ausführungsform nach Fig. 1 hat die Elektrodeneinheit 2 am Rahmen 10 mehrere Arme 12, und die Elektroden sind jeweils an den Enden dieser Arme 12 angeordnet. Die Elektrode 22 ist so positioniert, daß sie beim Ansetzen der Elektrodeneinheit vorderhalb des Ohres mit dem Kopf in Berührung kommt. Die beiden anderen, als Ableitelektroden dienenden Elektroden sind so angeordnet, daß sie beim Ansetzen der Elektrodeneinheit im Scheitelbereich bzw. hinterhalb des Ohres mit dem Kopf in Berührung gebracht werden.

Der Reizgeber ist bei der Ausführungsform nach Fig. 1 als Freifeldlautsprecher 30 dargestellt, der die umgesetzten Klickfolgen in den Raum abstrahlt.

Die Ausführungsform nach Fig. 2 zeigt zunächst eine mögliche Variante der Elektrodeneinheit 2. Dort sind, im Gegensatz zu der in den Fig. 1 und 2 dargestellten Ausführungsformen, alle Elektroden der Elektrodeneinheit etwa in einer Linie angeordnet, weshalb hier das Zentralstück 11 des Rahmens 10 keine seitwärts wegragenden Arme 12 benötigt. Die Bezugselektrode 22 ist dabei zwischen den beiden Ableiteelektroden 21 angeordnet. Die Elektroden können gefedert montiert sein.

Des weiteren verdeutlicht Fig. 2 mögliche Alternativen bezüglich des Reizgebers gegenüber Fig. 1. Das in Fig. 2 als Reizgeber bezeichnete Element 35 kann ein Einsteckhörer sein, der die Klick-Folge also direkt im Ohr erzeugt.

Eine weitere Alternative besteht darin, daß der als Einsteckhörer ausgebildete Reizgeber zugleich als Gehörgangelektrode dient, also auch mit zur Signalableitung anstelle einer der Elektroden 21 oder zusätzlich dazu dient.

Als weitere Alternative kann der in Fig. 2 als Element 35 nur schematisch dargestellte Reizgeber eine OAE-Sonde sein, also eine Sonde für otoakustische Emissionen. Eine solche Sonde enthält einen Lautsprecher sowie ein Mikrophon. Sie erzeugt, akustische Signale direkt im Ohr und misst, ebenfalls im Ohr, das akustische Echo, das die äußeren Haarzellen in der Hörschnecke produzieren. Die Verwendung einer OAE-Sonde als Reizgeber ermöglicht dabei in einem Zuge verschiedenartige Messungen.

Eine weitere, nicht eigens dargestellte Variante bezüglich des Reizgebers besteht in der Verwendung eines Knochenleitungshörers, wie er an sich bekannt ist.

Das Gehäuse des EEG-Verstärkers 40 ermöglicht bei allen Ausführungsformen zugleich das Ergreifen und Festhalten der Elektrodeneinheit 2 durch den Operator beim Gebrauch. Der Rahmen 10 bildet eine relativ starre Anordnung mit definierter gegenseitiger Lage der Elektroden zur Potentialableitung, wobei gleichzeitig jedoch eine gewisse Flexibilität gegeben ist. Es versteht sich von selbst, daß die Kontaktflächen der Elektroden bei Bedarf mit einem geeigneten Medium zur Verringerung des Übergangswiderstands zwischen Haut und Elektrode versehen werden können.

An dem Gehäuse des EEG-Verstärkers 40 bzw. am Rahmen 10 der Elektrodeneinheit ist zweckmäßigerweise auch eine Anzeige, beispielsweise eine Leuchtanzeige 5, vorgesehen, welche die Betriebsbereitschaft bzw. die Funktion des Geräts anzeigt, sowie ein fingerbetätigbarer Schalter 6 zum Auslösen der Klickfolgenerzeugung und des Messvorgangs durch das Gerät, wenn der Operator die Elektrodeneinheit am Kopf des Probanden hält.

In der Praxis wird man die Elektrodeneinheit in verschiedenen Standardgrößen zur Verwendung bei Säuglingen, Kindern bzw. erwachsenen Probanden ausbilden.

Anstelle lediglich des EEG-Verstärkers 40 kann auch die komplette Messeinrichtung für die abgeleiteten Gehirnpotentiale am Rahmen der Elektrodeneinheit 2 angeordnet sein. Die Signalübertragung zwischen der Elektrodeneinheit 2 und der Signalgeber/Auswerteeinheit 1 kann statt über die im Ausführungsbeispiel dargestellte Kabelverbindung auch drahtlos erfolgen. Auch die Auslösung der Klickreizfolge und der Messwerterfassung durch Betätigen eines Schalters an der Elektrodeneinheit kann auf drahtlosem Wege erfolgen.

## Patentansprüche

1. Gerät zur Ableitung akustisch evozierter Gehimpotentiale bei der Hirnstamm-Audiometrie, mit einer Anzahl von am Kopf des Probanden anzuordnenden Elektroden (21, 22), die mindestens eine Ableitungselektrode (21) und eine an einer davon entfernten Stelle am Kopf anzuordnende Bezugselektrode (22) umfassen, sowie mit einem elektroakustischen Wandler (30, 35) als Reizgeber zur akustischen Stimulation des Ohres wobei die Elektroden und der Wandler mit einer Signalgeber/Auswerteeinheit (1) verbunden sind, die den Wandler mit elektrischen Reizsignalen beaufschlagt und die von den Elektroden abgeleiteten Potentiale auswertet, **dadurch gekennzeichnet, daß** die Elektroden (21, 22) an einer Elektrodeneinheit (2) zusammengefasst sind, die einen relativ starren Rahmen (10, 11, 12) aufweist, der die Elektroden in definierter räumlicher Orientierung relativ zueinander haltert und das Halten der Elektrodeneinheit am Probandenkopf unter gleichzeitiger Anlage aller Elektroden an den entsprechenden Ableitungspunkten ermöglicht, daß weiter als elektroakustischer Wandler (30, 35) ein Freifeldlautsprecher, ein Einsteckhörer, eine einen Lautsprecher enthaltende Sonde für otoakustische Emissionen, oder ein Knochenleitungshörer dient.

2. Gerät nach Anspruch 1, **dadurch gekennzeichnet, daß** der Einsteckhörer gleichzeitig als Gehörgangelektrode ausgebildet ist, die zusätzlich zu den Elektroden an der Elektrodeneinheit als Ableitungs- oder Bezugselektrode dient.

3. Gerät nach Anspruch 1 oder 2, **dadurch gekennzeichnet, daß** die am Rahmen gehalterten Elektroden drei Elektroden umfassen, von welchen eine Elektrode (21) für eine Potentialableitung im Scheitelbereich vorgesehen und an einem entsprechenden Rahmenarm (12) angeordnet ist, eine zweite Elektrode (21) zur Potentialableitung an einem hinter dem Ohr gelegenen Kopfbereich angeordnet und die dritte Elektrode (22) als Bezugselektrode zur Anlage an einem vorderhalb des Ohres gelegenen Kopfbereich am Rahmen angeordnet ist.

4. Gerät nach einem der Ansprüche 1 bis 3, **dadurch gekennzeichnet, daß** die Elektroden (21, 22) an dem Rahmen (10, 11, 12) in einer etwa linienförmigen Anordnung angeordnet sind.

5. Gerät nach einem der Ansprüche 1 bis 4, **dadurch gekennzeichnet, daß** an dem Rahmen (10, 11, 12) auch ein EEG-Verstärker (40) angeordnet ist.

6. Gerät nach Anspruch 5, **dadurch gekennzeichnet, daß** der EEG-Verstärker (40) mit seinem Gehäuse als Griffstück für den Rahmen (10, 11, 12) ausgebildet ist.

7. Gerät nach Anspruch 5 oder 6, **dadurch gekennzeichnet, daß** auch eine komplette Messeinrichtung zum Messen der abgeleiteten Gehimpotentiale an dem Rahmen (10, 11, 12) angeordnet ist.

8. Gerät nach Anspruch 6 oder 7, **dadurch gekennzeichnet, daß** an dem Rahmen (10) auch eine Einrichtung zur drahtlosen Übertragung der erfassten Signale bzw. der Messwerte zu einer Signalgeber/Auswerteeinheit (1) vorgesehen ist.

9. Gerät nach einem der Ansprüche 1 bis 8, **dadurch gekennzeichnet, daß** an dem Rahmen (10) ein fingerbetätigbarer Schalter (6) zum Auslösen der Erzeugung akustischer Reizsignale und der Messwerterfassung vorgesehen ist.

## Claims

1. Device for the derivation of acoustically evoked cerebral potentials in brain stem audiometry, with a number of electrodes (20, 21) to be arranged on the head of the subject and comprising at least one derivation electrode (21) and, to be arranged at a point remote from this on the head, a reference electrode (22), and with an electro-acoustic transducer (30, 35) serving as an exciter for acoustic simulation of the ear, where the electrodes and the transducer are connected to a transducer sensor/evaluation unit (1) which supplies the electrical stimulus signals to the transducer and evaluates the potentials derived by the electrodes, **characterised in that** the electrodes (21, 22) are grouped together in an electrode unit (2) which has a relatively rigid frame (10, 11, 12) which holds the electrodes in defined spatial orientation relative to each other and allows the holding of the electrode unit at the subject's head during simultaneous application of all electrodes to corresponding derivation points, and **in that** a free field speaker, a plug-type earpiece, a sensor containing a speaker for otoacoustic emissions or a bone conductance earpiece is used as an electro-acoustic transducer (30, 35) .

2. Device according to claim 1, **characterised in that** the plug-type earpiece is formed simultaneously as an auditory canal electrode which serves as a derivation or reference electrode in addition to the electrodes on the electrode unit.

3. Device according to claim 1 or 2, **characterised in that** the electrodes mounted on the frame comprise three electrodes of which one electrode (21) is provided for potential derivation in the crown area and is arranged on a corresponding frame arm (12), a second electrode (21) is located for potential derivation at an area of the head located behind the ear, and the third electrode (22) as a reference electrode is arranged on the frame for location at a head area located in front of the ear.

4. Device according to one of claims 1 to 3, **characterised in that** the electrodes (21, 22) are arranged on the frame (10, 11, 12) in an approximately linear arrangement.

5. Device according to one of claims 1 to 4, **characterised in that** also arranged on the frame (10, 11, 12) is an EEG amplifier (40).

6. Device according to claim 5, **characterised in that** the EEG amplifier (40) is designed with its housing as a grip for the frame (10, 11, 12).

7. Device according to claim 5 or claim 6, **characterised in that** a complete measuring apparatus for measuring the derived cerebral potentials is also arranged on the frame (10, 11, 12).

8. Device according to claim 6 or 7, **characterised in that** a device for wireless transmission of the detected signals or measured values to a transducing sensor/evaluation unit (1) is also provided on the frame (10).

9. Device according to one of claims 1 to 8, **characterised in that** a finger-operable switch (6) for triggering the generation of acoustic stimulus signals and measured value acquisition is provided on the frame (10).

## Revendications

1. Dispositif pour la dérivation de potentiels cérébraux acoustiquement provoqués lors de l'audiométrie du tronc cérébral, comportant un nombre d'électrodes (21, 22) à disposer sur la tête du sujet, qui comportent au moins une électrode de dérivation (21) et une électrode de référence (22) à disposer à un endroit éloigné sur la tête par rapport à la première, ainsi qu'un convertisseur électroacoustique (30, 35) en tant qu'excitateur pour la stimulation acoustique de l'oreille, les électrodes et le convertisseur étant connectés à un émetteur de signaux/une unité de dépouillement (1) qui envoie des signaux d'excitation électriques au convertisseur et dépouille les potentiels dérivés par les électrodes, **caractérisé en ce que** les électrodes (21, 22) sont réunies sur un ensemble d'électrodes (2) qui présente un cadre (10, 11, 12) relativement rigide, lequel maintient les électrodes avec une orientation définie dans l'espace les unes par rapport aux autres et permet de tenir l'ensemble d'électrodes sur la tête du sujet, avec application simultanée de toutes les électrodes aux points de dérivation correspondants, **en ce qu'**en outre le convertisseur électroacoustique (30, 35) est constitué d'un haut-parleur à champ libre, d'un écouteur à insertion, d'une sonde pour émissions otoacoustiques comportant un haut-parleur ou d'un vibreur à conductions par les os.

2. Dispositif selon la revendication 1, **caractérisé en ce que** l'écouteur à insertion est en même temps agencé sous forme d'électrode de conduit auditif qui sert d'électrode de dérivation ou de référence, en supplément aux électrodes de l'ensemble d'électrodes.

3. Dispositif selon la revendication 1 ou 2, **caractérisé en ce que** les électrodes fixées au cadre comportent trois électrodes dont une électrode (21) est prévue pour la dérivation de potentiels dans la zone du sommet et est disposée sur un bras de cadre (12) correspondant, une deuxième électrode (21) est disposée pour la dérivation des potentiels dans une zone de la tête située derrière l'oreille et la troisième électrode (22) est disposée sur le cadre en tant qu'électrode de référence pour être placée dans une zone de la tête située devant l'oreille.

4. Dispositif selon une des revendications 1 à 3, **caractérisé en ce que** les électrodes (21, 22) sont disposées sur le cadre (10, 11, 12) dans une disposition approximativement linéaire.

5. Dispositif selon l'une quelconque des revendications 1 à 4, **caractérisé en ce qu'**un amplificateur EEG (40) est également disposé sur le cadre (10, 11, 12).

6. Dispositif selon la revendication 5, **caractérisé en ce que** l'amplificateur EEG (40) avec son boîtier est formé en tant que poignée pour le cadre (10, 11, 12).

7. Dispositif selon la revendication 5 ou 6, **caractérisé en ce qu'**un dispositif de mesure complet pour mesurer les potentiels cérébraux dérivés est également disposé sur le cadre (10, 11, 12).

8. Dispositif selon la revendication 6 ou 7, **caractérisé en ce qu'**un dispositif pour la transmission sans fil des signaux et des valeurs de mesure saisis à un émetteur de signaux/une unité de dépouillement (1) est également prévu sur le cadre (10).

9. Dispositif selon l'une quelconque des revendications 1 à 8, **caractérisé en ce qu'**un interrupteur (6) pouvant être actionné par un doigt pour déclencher la production de signaux d'excitation acoustiques et la saisie des valeurs de mesure est prévu sur le cadre (10).
